# EUROPEAN PATENT APPLICATION

(11) **EP 2 532 368 A1**
(43) Date of publication of application: **12.12.2012**
(21) Application number: 11169113.5
(22) Date of filing: 08.06.2011
(51) Int. Cl.: A61K 47/48, A61P 43/00

(54) **"Composition for oral delivery of biologically-active substances"**

(71) Applicant: Peluso, Gianfranco, 82100 Benevento (IT); D'Argenio, Giuseppe, 80144 Napoli (IT); Caporaso, Nicola, 82030 Cautano (BN) (IT)
(72) Inventor: Peluso, Gianfranco, 82100 Benevento (IT); D'Argenio, Giuseppe, 80144 Napoli (IT); Caporaso, Nicola, 82030 Cautano (BN) (IT)
(74) Representative: Banfi, Paolo

(57) **Abstract**

The present invention relates to compositions and methods for oral delivery of biologically-active substances. More particularly, the invention relates to systems and methods for treating inflammatory diseases, such as inflammatory bowel disease, by means of particulate carriers loaded with therapeutic agents, which are targeted to cell receptors present on immune-competent cells.

## Description

### BACKGROUND

### Inflammatory Bowel Disease

Inflammatory bowel disease (IBD) is a multifactorial disease with probable genetic heterogeneity [D.C. Baumgart, and S.R. Carding. Inflammatory bowel disease: cause and immunobiology. The Lancet. 2007 369:1627-1640]. In addition, several environmental risk factors contribute to its pathogenesis. Worldwide, the incidence rates for Ulcerative Colitis (UC) vary from 0.5 to 24.5 per 100,000 person-years. The classical presentation is that of rectal bleeding and diarrhea, with other symptoms such as urgency, tenesmus and abdominal cramping being also common. The disease may be limited to the rectum or extend proximally to include total colitis and is characterized by a remission-relapse course in most patients.

It is now widely accepted that inflammatory bowel disease results from an inappropriate response of a defective mucosal immune system to the indigenous flora and other luminal antigens. Experimental evidence from studies in vitro, in animals, and in human beings suggests that several, not mutually exclusive, pathways might result in inflammatory cascades.

First, people with inflammatory bowel disease have disturbed innate immune mechanisms of the epithelial layer. In these people, mucosal epithelial cells have a different pattern of toll-like receptors (TLR) expression. One of the most important pattern-recognition receptors families in mammals are toll-like receptors (TLR), first described in the fruitfly, [Akira S, Takeda K. Toll-like receptor signalling. Nat Rev Immunol 2004; 4: 499-511; Iwasaki A, Medzhitov R. Toll-like receptor control of the adaptive immune responses. Nat Immunol 2004; 5: 987-95.] The ten to 15 TLR (dependent on the species) identified so far are individually specialised, but taken together, are probably capable of recognising most microbe associated molecular patterns. On recognition of their specific microbe-associated molecular patterns, TLR trigger innate and adaptive antimicrobial responses in an intracellular signalling process that culminates in the activation of the transcription factor nuclear factor kappa B (NFκB) and induction of the inflammatory cytokine cascade, [Rakoff -Nahoum S, Paglino J, Eslami-Varzaneh F, Edberg S, Medzhitov R. Recognition of commensal microflora by toll-like receptors is required for intestinal homeostasis. Cell 2004; 118: 229-41; Cario E, Gerken G, Podolsky DK. Toll-like receptor 2 enhances ZO-1-associated intestinal epithelial barrier integrity via protein kinase C. Gastroenterology 2004; 127: 224-38.] In the absence of pathogens, TLR interactions with commensals have been shown to contribute to intestinal homoeostasis and maintenance of an intact epithelial barrier [Girardin SE, Boneca IG, Carneiro LA, et al. Nod1 detects a unique muropeptide from gram-negative bacterial peptidoglycan. Science 2003; 300: 1584-87; Girardin SE, Boneca IG, Viala J, et al. Nod2 is a general sensor of peptidoglycan through muramyl dipeptide (MDP) detection. J Biol Chem 2003; 278: 8869-72].

Whereas healthy intestinal epithelial cells constitutively express TLR3 and TLR5 basolaterally, TLR2, and TLR4 are usually barely detectable. By contrast, TLR4 is strongly upregulated in active Crohn's disease, and in UC [Cario E, Podolsky DK. Differential alteration in intestinal epithelial cell expression of toll-like receptor 3 (TLR3) and TLR4 in inflammatory bowel disease. Infect Immun 2000; 68: 7010-17]. Intestinal epithelial cells also express TLR9, which enables them to directly respond to bacterial DNA, resulting in secretion of interleukin 8, which is a granulocyte chemoattractant [Akhtar M, Watson JL, Nazli A, McKay DM. Bacterial DNA evokes epithelial IL-8 production by a MAPK-dependent, NF-kappaB independent pathway. FASEB J 2003; 17: 1319-21].

Second, antigen recognition and processing by professional antigen-presenting cells is disturbed in people with inflammatory bowel disease. Animal and in-vitro studies suggest that dendritic cells incorrectly recognise commensal bacteria and induce a Th1 and possibly Th17, proinflammatory immune responses normally directed at pathogens. This could be due to dysfunctional or exaggerated pattern-recognition receptor responses [Franchimont D, Vermeire S, E1 HH, et al. Defi cient host-bacteria interactions in inflammatory bowel disease? The toll-like receptor (TLR)-4 Asp299gly polymorphism is associated with Crohn's disease and ulcerative colitis. Gut 2004; 53: 987-92]. Human intestinal dendritic-cell populations in inflammatory bowel disease are insufficiently characterised, mainly owing to the scarcity of highly specific antibodies and their low numbers. Recently, it has been shown an increased frequency of mature-ie, activated-dendritic cells in the inflamed mucosa of patients with active inflammatory bowel disease [Hart AL, Al-Hassi HO, Rigby RJ, et al. Characteristics of intestinal dendritic cells in infl ammatory bowel diseases. Gastroenterology 2005; 129: 50-65, Baumgart DC, Metzke D, Wiedenmann B, Dignass AU. Activated dendritic cells are significantly increased in inflamed intestinal mucosa of inflammatory bowel disease patients. Gastroenterology 2004; 126: A159] Human dendritic cells from inflammatory bowel disease patients express gut homing markers and also showed an aberrant response to microbial surrogate stimuli like CpG-DNA and lipopolysaccharide [Baumgart DC, Metzke D, Schmitz J, et al. Patients with active infl ammatory bowel disease lack immature peripheral blood plasmacytoid and myeloid dendritic cells. Gut 2005; 54: 228-36]. This absence of the regulatory capacity of dendritic cells might also contribute to the repeated activation of certain (gut homing) memory T cells or failure to delete these over reactive T-cell populations (absence of peripheral tolerance), thereby perpetuating inflammation [Steinman RM, Nussenzweig MC. Avoiding horror autotoxicus: the importance of dendritic cells in peripheral T cell tolerance. Proc Natl Acad Sci USA 2002; 99: 351-58; Papadakis KA, Zhu D, Prehn JL, et al. Dominant role for TL1A/DR3 pathway in IL-12 plus IL-18-induced IFN-gamma production by peripheral blood and mucosal CCR9+ T lymphocytes. J Immunol 2005; 174: 4985-90].

### IBD Treatment

Biological therapies have radically changed the management of IBD but must be used judiciously with great awareness of possible adverse events. For example, newer targeted treatments such as anti-tumor necrosis factor (TNF) agents are effective in a subset of patients but have to be administered systemically and also are associated with significant side effects [Ballinger A., Adverse effects of nonsteroidal anti-inflammatory drugs on the colon, Curr Gastroenterol Rep, Volume: 10, (2008), pp. 485-489]. In addition, although widely heralded for their efficacy, these therapies have also stirred controversy over the potential economic impact that they will have upon the world's health-care systems.

5-Aminosalicylic acid (5-ASA; mesalazine) remains the mainstay treatment in mild to moderate IBD [Travis SPL, Stange EF, Leimann M, Øresland T, BemelmanWA, Chowers Y, et al. European evidence-based consensus on the management of ulcerative colitis: current management. J Crohn Colitis 2008;2:24-62]. Although the exact mechanism for mesalazine's anti-inflammatory effect is largely unknown, it appears to act locally on the colonic mucosa and reduces the inflammation associated with UC through modulating a broad range of inflammatory processes [Prakash A, Markham A. Oral delayed-release mesalazine: a review of its use in ulcerative colitis and Crohn's disease. Drugs 1999;57(3):383-408]. Mesalazine has been shown to inhibit the production of mediators of the lipoxygenase (e.g., leukotriene B4) and cyclo-oxygenase (e.g., prostaglandin [PG] E2 and PGD2) pathways, as well as platelet-activating factor, which are elevated in the colonic tissue of patients with inflammatory bowel disease. In addition, mesalazine inhibits interleukin (IL)-1 and IL-2 production and/or release in colonic epithelial cells as well as inhibits a number of other cytokines, including tumor necrosis factor (TNF)-α and operates as a potent antioxidant and free-radical scavenger. Furthermore, it prevents injury induced by reactive oxygen species, potentially involved in the pathogenesis of inflammatory bowel disease.

The nuclear transcription factor NF-κB may play an important role in bowel inflammation, since it regulates the transcription of genes for a wide variety of proinflammatory molecules, including cytokines, adhesion molecules and oxidants, and is elevated in the mucosa of patients with UC, but not in non-inflamed mucosa [Bantel H, Berg C, Vieth M, Stolte M, Kruis W, Schulze-Osthoff K. Mesalazine inhibits activation of transcription factor NF-κB in inflamed mucosa of patients with ulcerative colitis. Am J Gastroenterol 2000;95(12):3452-7]. At the molecular level, mesalazine has also been recognized as an inhibitor of NF-κB in epithelial cells and macrophages in the mucosa and an agonist of the peroxisome proliferator-activated receptor (PPAR)-γ [Rousseaux C, Lefebvre B, Dubuquoy L, Lefebvre P, Romano O, Auwerx J, et al. Intestinal anti-inflammatory effect of 5-aminosalicylic acid is dependent on peroxisome proliferator-activated receptor-gamma. J Exp Med 2005;201:1205-15]. These receptors are expressed at particularly high levels in colon epithelial cells, where their expression appears to be at least in part stimulated by gut bacteria.

Current hypothesis is that 5-aminosalicylic acid is believed to act by activating a class of nuclear receptors, most importantly PPAR-γ, involved in the control of inflammation, cell proliferation, apoptosis and metabolic function, e.g., modulation of inflammatory cytokine production, modulation of Re1A/p65 dephosphorylation, leading to decreased transcriptional activity of NF- B, and reduced synthesis of prostaglandins and leukotrienes [Desreumaux P, Ghosh S. Review article: mode of action and delivery of 5-aminosalicylic acid-new evidence. Aliment Pharmacol Ther 2006;24(Suppl.1):2-9]. Activation of PPAR-γ also has anti-tumorigenic effects which manifest as anti-proliferative activities, pro-differentiation activities, pro-apoptotic activities, inhibition of the formation of aberrant crypts foci and possibly inhibition of the development of colon tumors. At least PPAR-γ has been shown to suppress tumor formation through a tumor-suppressor gene at an early stage in the development of colon tumors, thereby interfering with the Wnt/b-catenin signalling pathway [Osawa E, Nakajima A, Wada K, Ishimine S, Fujisawa N, Kawamori T, et al. Peroxysome proliferator-activated receptor gamma ligands suppress colon carcinogenesis induced by azoxymethane in mice. Gastroenterology 2003;124:361-7].

In left-sided and extensive cases, a combination of oral and topical mesalazine appears to be more effective than either alone; however, this is probably not simply a dose-response effect, as higher topical 5-ASA doses do not improve efficacy [Safdi M, DeMicco M, Sninsky C, Banks P,Wruble L, Deren J, et al. A double-blind comparison of oral versus rectal mesalamine versus combination therapy in the treatment of distal ulcerative colitis. Am J Gastroenterol 1997;92:1867-71]. A number of oral 5-ASA agents are commercially available, including azo-bond prodrugs such as sulfasalazine, olsalazine and balsalazide, and delayed- and controlled-release forms of mesalazine. In the most recent meta-analysis, mesalazine is shown to be as effective as sulfasalazine for inducing response or remission (OR=0.83, 95%CI: 0.60-1.13) [Sutherland L, MacDonald JK. Oral 5-aminosalicylic acid for induction of remission in ulcerative colitis. Cochrane Database Syst Rev 2006;2:CD000543], and is better tolerated. Somewhat in contrast, the use of sulfasalazine is mainly limited by its side effects (including nausea, vomiting, abdominal pain, fever, skin rash, agranulocytosis, neutropenia, male infertility, folate deficiency, neuropathy, autoimmune hemolysis and rarely nephrotoxicity, hepatotoxicity or pancreatitis) and the high rate of intolerance (up to 20%). Mild side effects are more common with sulfasalazine, while some of the more severe side effects, e.g., pancreatitis is more common with mesalazine (OR: 7.0) and intestinal nephritis was exclusively described for mesalazine [Ransford RA, Langman MJ. Sulphasalazine and mesalazine: serious adverse reactions re-evaluated on the basis of suspected adverse reaction reports to the committee on safety of medicines. Gut 2002;51:536-9]. Of note, in a recent review by the Cochrane group, mesalazine was not superior compared to sulfasalazine for inducing response or remission (OR = 0.83, 95%CI: 0.60-1.13) [Sutherland L, MacDonald JK. Oral 5-aminosalicylic acid for induction of remission in ulcerative colitis. Cochrane Database Syst Rev 2006;2:CD000543], but was better tolerated.

Much emphasis has been placed on how different delivery systems may influence response to 5-ASAs; however, evidence in clinical practice for variability in efficacy is rather weak. Delivery systems can be divided into azo-compounds, controlled release, pH-dependent (either pH 6 or 7) and composite (pH-dependent combined with controlled release) [Desreumaux P, Ghosh S. Review article: mode of action and delivery of 5-aminosalicylic acid-new evidence. Aliment Pharmacol Ther 2006;24(Suppl.1):2-9]. A new oral delayed-release formulation of mesalazine utilizing Multi-Matrix System (MMX) technology (hereafter referred to as MMX mesalazine) was recently approved in the US for the induction and maintenance of remission in patients with active, mild to moderate UC [McCormack PL, Robinson DM, Perry CM. Delayed-release multi-matrix system (MMX™) mesalazine in ulcerative colitis. Drugs 2007;67:2635-42]. It is a high dose (mesalazine 1.2 g/tablet), delayed-release form that permits once daily administration. The MMX technology involves incorporating mesalazine into a lipophilic matrix, which is itself dispersed within a hydrophilic matrix, to delay and prolong dissolution. A gastro-resistant polymer film prevents initial drug release until exposed to a pH <7, thus the film coat normally starts to dissolve only in the terminal ileum. The hydrophilic matrix is then exposed to intestinal fluids and swells, resulting in the formation of a viscous gel mass with a slow and gradual release of mesalazine throughout the length of the colon.

However, in the future even more effective drug delivery based on 5-ASA are expected to increase the local effects and to decrease the systemic side effects.

### Delivery systems

A major advance in therapeutic strategies in diseases such as IBD would be the ability to target drugs to the site of inflammation in sufficient quantities to maximize local drug concentration and minimize systemic side effects. However, targeting drugs to the site of inflammation has remained a challenge in IBD because of the lack of vehicles that could carry sufficient drugs or that could be released at the site of inflammation. Another problem is created by organs of the gastrointestinal tract, particularly the colon, because it is a challenge to deliver the drug to the colon with minimal digestive enzyme degradation and/or systemic absorption [Hebrard G., Hoffart V., Cardot J., et al. Investigation of coated whey protein/alginate beads as sustained release dosage form in simulated gastrointestinal environment, Drug Dev Ind Pharm, 2009, 14:1-10]. Various carriers have been designed to release the drug at a specific pH value, to be resistant to digestive enzymes, and/or require bacterial cleavage for activation, and several of these carriers currently are being investigated [Lamprecht A., Yamamoto H., Takeuchi H., et al. A pH-sensitive microsphere system for the colon delivery of tacrolimus containing nanoparticles, J Control Release, 2005, 104:337-346; Lamprecht A., Yamamoto H., Takeuchi H., et al. Nanoparticles enhance therapeutic efficiency by selectively increased local drug dose in experimental colitis in rats, J Pharmacol Exp Ther, 2005, 315:196-202; Leserman L.D., Machy P., Barbet J., Cell-specific drug transfer from liposomes bearing monoclonal antibodies, Nature, 1981, 293:226--228] However, most of these drugs need to be administered in large doses, multiple times a day, resulting in poor patient compliance.

Among the various carriers proposed for drug delivery, polymeric nanoparticles (NPs) have been studied for several decades. The most widely used polymers to engineer NPs are aliphatic polyesters such as polylactide (PLA) [Laroui H., Grossin L., Leonard M., et al. Hyaluronate-covered nanoparticles for the therapeutic targeting of cartilage, Biomacromolecules, 2007, 8:3879-3885], polyglycolide, and co-polymers, [Makhlof A., Tozuka Y., Takeuchi H., pH-sensitive nanospheres for colon-specific drug delivery in experimentally induced colitis rat model, Eur J Pharm Biopharm, 2009, 72:1-8; Meissner Y., Pellequer Y., Lamprecht A., Nanoparticles in inflammatory bowel disease: particle targeting versus pH-sensitive delivery, Int J Pharm, Volume:, 2006, 316:138--143] which have been approved by the US Food and Drug Administration and have well-characterized biocompatibility and (bio)degradability properties [Feng S.S., Mei L., Anitha P., et al. Poly(lactide)-vitamin E derivative/montmorillonite nanoparticle formulations for the oral delivery of Docetaxel, Biomaterials, 2009, 30:3297-3306; Yang H., Li K., Liu Y., et al. Poly(D,L-lactide-co-glycolide) nanoparticles encapsulated fluorescent isothiocyanate and paclitaxol: preparation, release kinetics and anticancer effect, J Nanosci Nanotechnol, 2009, 9:282-287].

Recent studies have been reported using nanotechnology for cell targeting. Thus, micelles have been described as promoting the uptake of 40-nm colloidal gold particles, particularly in the rectal area [Fukui H., Murakami M., Yoshikawa H., et al. Studies on the promoting effect of lipid-surfactant mixed micelles (MM) on intestinal absorption of colloidal particles: Dependence on particle size and administration site, J Pharmacobiodyn, 1987, 10:236--242].

### Summary of invention

The present invention provides a composition comprising a vector particle (herein also referred to as "carrier particle") encapsulating an active agent, said vector particle being attached through a spacer molecule to a binding moiety that binds to a target molecule present in mammalian immunocytes such as the dendritic cells present in intestinal tissue. The target molecule is selected from cell molecules which promote accumulation and retention of the carrier particle in the target cells. The vector particle comprises a protective matrix which is suitable for encapsulating or otherwise retaining (e.g. by absorption or dispersion) an active agent therein. Useful binding moieties are selected to specifically bind to cell receptors present in cells which promote inflammatory and/or immunological response.

The invention therefore provides a composition for oral administration comprising:
a) a vector particle consisting of a material which resists proteolytic degradation, wherein said particle encapsulates or retains an active agent;
b) a binding moiety attached to said vector particle, wherein said binding moiety is able to specifically bind to cell receptors present in target cells which promote inflammatory and/or immunological response; and
c) a spacer molecule linking the vector particle and the binding moiety.

The composition may further comprise a coating protective matrix suitable for coating the vector particle attached to the binding molecule, said coating protective matrix being able to resist proteolytic degradation.

The binding moiety will bind specifically to a target molecule present in a specific cell, such as intestinal dendritic cell, with sufficient affinity or avidity.

The target molecule will preferably be an inflammatory and/or immune-response promoting receptor such as, for example, the Toll-like receptors. The binding moiety may be an antibody or fragment thereof, or specific aptamers or their derivatives able to bind said receptor.

A pharmaceutical preparation according to the present invention comprises a vector-particle delivery system as described above in a pharmaceutically acceptable vehicle.

Methods for systematically delivering an active agent to a mammalian host according to the present invention comprise administering to the host a composition including an active agent in a carrier particle attached through a spacer molecule to a binding moiety which binds specifically to an inflammatory- and/or immune-response promoting target molecule present in mammalian cells, preferentially immune-competent cells. The carrier particle will be absorbed by the target cell prior to release of the active agent from the particle. These methods are particularly useful for delivering drugs and other active agents which are sensitive to degradation in the intestine, are normally poorly absorbed in the intestine or must carry out a therapeutic action preferentially at local level in the intestine.

### Description of specific embodiments

The composition and methods of the present invention are intended for targeted active agent delivery to inflammatory and/or immune cells where the active agents will carry out their therapeutic effect. The active agents are present in carrier particles which are attached through a spacer molecule to binding moieties which recognize certain conserved receptors in the immune cells present in inflammatory tissue. The active agent is released from the carrier once inside the cell.

The terms "active agent" and "drug" are used interchangeably herein. The active agents that can be delivered according to the present invention include inorganic and organic drugs without limitation and include drugs that act on gastro-intestinal system as well as on immunological system. The active drug that can be delivered for acting on these recipients includes, but is not limited to, anti-inflammatories, immunomodulants, biocides, bactericides, antiviral agents, probiotics, nutraceuticals, enzymes, cytoprotectants, vaccines, polynucleotides, polypeptides, polysaccharides, and the like. In a preferred embodiment the active agent is 5-aminosalicylic acid (5-ASA).

The present invention is particularly suitable for delivering drugs to target cells, such as intestinal immune cells, with the purpose to avoid the systemic side effects of the drugs. The active agent for delivery in this invention can be in various pharmaceutically acceptable forms, such as uncharged molecules, molecular complexes, and pharmacologically acceptable salts. Derivatives such as esters, ethers and amides, can be used. The conserved receptors (target molecules) will usually be toll like receptors (TLR) which are naturally present in mammalian immune cells while only TLR9 is present also in mammalian enterocytes. Suitable binding moieties will comprise chemical structures which are capable of binding to the target molecule(s) with sufficient affinity or avidity to promote the accumulation of the active compound in the cells. By affinity it is meant that a single binding moiety will bind to a single target molecule with a minimum threshold binding constant of 10⁻⁵M or less, preferably less than 10⁻⁶M and more preferably less than 10⁻⁷M. By "avidity", it is meant that two or more binding moieties present on a single drug carrier particle will bind to two or more separate target molecules in immune cells, where the individual binding interactions between binding moieties and target molecules contribute to an overall binding constant of 10⁻⁵M or less, preferably less than 10⁻⁶M and more preferably less than 10⁻⁹M. It will be appreciated that the individual binding constants of each binding interaction may be higher than those set forth, but that taken into the aggregate, binding between the binding moiety and target molecule will preferably meet these threshold levels in order to promote the desired accumulation of the drug carrier particles by the immunocyte.

The binding moieties may be any small or large molecular structure which provides the desired binding interaction(s) with the cell receptors in immunocytes, such as antibodies, aptamers or fragments and derivatives thereof.

Conveniently, the binding moieties will be aptamers able to mimic natural ligands for TLR. The binding moiety may comprise a variety of molecular structures, including nucleic acids and other receptor ligands, and fragments thereof. It is possible to prepare and/or synthesize other molecules which are capable of binding the target with the requisite affinity or avidity. For example, ligands for TLR9, including oligodeoxynucleotides containing unmethylated CpG motifs and synthetic immune modulatory oligonucleotides, may be produced using conventional techniques, as described by Putta et al. [Putta M.R., Yu D., Bhagat L., Wang D., Zhu F-G., and Kandimalla E.R. Impact of Nature and Length of Linker Incorporated in Agonists on Toll-Like Receptor 9-Mediated Immune Responses. 2010, 53: 3730-3738].

Additionally, it is possible to design other non-protein compounds to be employed as the binding moiety, using techniques known to those working in the area of drug design. Such methods include, but are not limited to, self-consistent field (SCF) analysis, configuration interaction (CI) analysis, and normal mode dynamics computer programs, all of which are well described in the scientific literature (see Rein et al., Computer-Assisted Modelling of Receptor-Ligand Interactions, Alan Liss, New York, 1989).

The vector particle of the present invention is based on a matrix which encapsulates or otherwise retains (e.g. by absorption or dispersion) the drug therein. The structure of the matrix will be such that (1) the particle can be covalently or non-covalently attached to the binding moiety through a spacer molecule, as described below, (2) the particle will protect the drug from degradation while present in the stomach and intestines, and (3) the particle will release the drug at a desired time, after the particle has been absorbed by the immunocyte. The carrier particle will be composed of a material which resists enteric degradation. The carrier particle may comprise a variety of forms, including metal particles and sols, ceramic particles, protein structures, carbohydrate structures, and the like, but will preferably comprise organic polymers such as polyethylene glycol (PEG), poly(lactic-co-glycolic acid) (PLGA), poly-DL-lactide (PDLLA) (carrier polymers) which have been synthesized to coat or contain the drug, e.g. by absorption or dispersion. Such methods, generally referred to as encapsulation, are well described in the scientific and patent literature (see, for example, Heller 1980, Biomaterials 1:51 and Sidman et al. 1980, J Membrane Sci 1:277). Nanoparticles were formulated as enteric microcapsules for improved delivery to the intestine using the polymers cellulose acetate phthalate (CAP) and ethyl cellulose (EC). Interpenetrating polymer network (IPN) microcapsules of gellan gum and egg albumin were prepared by ionotropic gelation and covalent crosslinking method.

The spacer is an organic molecule capable of linking the matrix carrier polymer to the binding moiety by means of covalent or non covalent bonds. It is able to optimize the binding capacity of the binding moiety to the target molecule decreasing the steric hindrance of the vector particle. In a preferred embodiment the spacer is a polyamine which is covalently linked to the matrix carrier polymer. Most preferred polyamines are spermine and tetraethylenepentamine. It was surprisingly found that the presence of a spacer molecule as defined above is essential for the binding of the vector particle to the target molecules, by decreasing the steric hindrance of the backbone polymer.

The vector particle attached to the binding molecule may be coated with a suitable coating protective matrix able to resist proteolytic degradation. Preferably such protective matrix comprises chitosan, sodium alginate or mixtures thereof. More preferably, the coating protective matrix comprises a mixture of sodium alginate and chitosan in a 7/3 weight ratio.

Preferred embodiments of the invention are now further illustrated by the following examples.

### Example 1

### Synthesis of polyaminated-nanoparticles (NPs)

Active ester method was used to attach pendant polyamine groups to biocompatible material such as polyesters (i.e., poly(d,1-lactide-co-glycolide, PLGA) by the amide bond formation using coupling agents (i.e., N,N-dicylcohexyl carbodiimide, DCC/N-hydroxysuccinimide, NHS). In the process, PLGA, DCC, and NHS are dissolved in anhydrous dimethylformamide (DMF) and stirred for different periods of time at room temperature. To the final solution, polyamines such as spermine (SP) or tetraethylenepentamine (TP) are added and allowed to react for different periods of times at room temperature under magnetic stirring.

The polymer product SP-PLGA or TP-PLGA is isolated by precipitation into excess diethyl ether under vigorous stirring and is dissolved in methanol, followed by dialysis against excess deionized distilled water at 4°C. This procedure is repeated twice. The nanopartilces are prepared by different methods such as nanoprecipitation. The nanoparticulated samples are then freeze-dried and stored at -20°C under nitrogen atmosphere.

### Nanoparticles characterization: Dynamic light scattering (DLS) and Zeta potential measurements

The hydrodynamic diameter of the NPs is determined by dynamic light scattering (DLS) measurements using Zetasizer, Nano ZS (Malvern instruments, UK) employing a nominal 5 mW HeNe laser operating at 633 nm wavelength. The scattered light is detected at 173° angle. The refractive index (1.33) and the viscosity (0.89) of ultrapure water are used at 25°C for measurements. Measured sizes are presented as the average value of 20 runs. For zeta potential measurements, NPs are suspended in 1 ml 10 mM Tris, pH 7.4, followed by sonication. Zeta potential measurements are carried out in automatic mode and the values are presented as the average value of 30 runs. The Smoluchowski approximation was used to calculate zeta potential from the electrophoretic mobility.

### Synthesis of specific aptamers (APT)

Aptamers are single-stranded RNA or DNA molecules isolated through *an in vitro* selection process. Using a novel molecular evolution strategy that combines immunoprecipitation (IP) with systematic evolution of ligands by exponential enrichment, we have developed a selection method to facilitate the screening of high-affinity aptamers for the Toll-like receptors such as TLR 9. In preferred embodiments, the aptamer synthesis is carried out using TLR9 attached to a solid support. A candidate mixture of single stranded RNA molecules is then contacted with the solid support. In especially preferred embodiments, the single stranded RNA molecules have a 2'-fluoro modification on C and U residues, rather than a 2'-OH group. After incubation for a predetermined time at a selected temperature, the solid support is washed to remove unbound candidate nucleic acid ligand. The nucleic acid ligands that bind to the TLR protein are then released into solution, then reverse transcribed by reverse transcriptase and amplified using the Polymerase Chain Reaction. The amplified candidate mixture is then used to begin the next round of the selection process. In the above embodiments, the solid support can be a nitrocellulose filter. Nucleic acids in the candidate mixture that do not interact with the immobilized TLR can be removed from this nitrocellulose filter by application of a vacuum. In other embodiments, the TLR target is adsorbed on a dry nitrocellulose filter, and nucleic acids in the candidate mixture that do not bind to the target are removed by washing in buffer. In other embodiments, the solid support is a microtiter plate comprised of, for example, polystyrene. All ligands used had undetectable endotoxin levels (> 0.1 U/ml). Examples of aptamers are the following: single-stranded PO R10-60 (TLR-9 aptamer, 5'-CCA GTC GTA CAG GAA ACA TGC GTT CTA GAT GTT CGG GGC-3'), PO R10-5 (aptamer without CpG, 5'-CCA GCC ACC TAC TCC ACC AGT GCC AGG ACT GCT TGA GGG G-3'), PS-modified 1018 (5'-TGA CTG TGA ACG TTC GAG ATG A-3') and PS-1019 (ODN without CpG, 5'-TGA CTG TGA AGG TTG GAG ATG A-3'). Aptamer mutants were constructed by PCR mutagenesis using mutagenic primers and template DNA. The DNA sequences of these mutants were confirmed by an Applied Biosystems model 3100 automatic sequencer.

### Functionalization of polyaminated-nanoparticles with aptamers (NPs-APT)

NPs suspension in double-distilled water are incubated with coupling agents, such as NHS and 1-(3-dimethylaminopropyl)-3-ethylcarbodimide hydrochloride (EDC), for different periods of times. The resulting NHS-activated particles are covalently linked to aptamers (APT). The resulting nanoparticles (NPs-APT) are characterized by NMR and/or solid phase NMR to demonstrate the binding of aptamer moiety to the NPs surfaces.

### Affinity of polyamine-functionalysed nanoparticles to TLR

Aptamers are fragments of oligonucleotides which can bind specifically to target proteins in the presence of appropriate conformational change [Liao W, Randall BA, Alba NA, Cui XT (2008) Analytical and Bioanalytical Chemistry 392:861-864] but they have also the advantages of simpler screening methods, higher stability than antibodies and reusability.

The affinity of polyamine-functionalized nanoparticles to TLR is assessed using a combination of an electrochemical method [Xiao Y, Lai RY, Plaxco KW (2007) Nature Protocols 2:2875-2880; Wei F, Sun B, Guo Y, Zhao XS (2003) Biosens Bioelectron 18:1157-1163] with enzymatic amplification and an aptamer probe attached on the nanoparticles that undergoes target-induced conformational change once bound to the TLR.

In this assay, the binding of TLR to aptamer generates signals by the conformational change of the aptamer. Correct folding of the aptamer allows high-affinity binding between the aptamer and TLR; misfolding and non-folding result in low signal-to-noise ratios (SNRs) [Chen CL, Wang WJ, Wang Z, Wei F, Zhao XS (2007) Nucleic Acids Res 35:2875-2884]. In addition, the specific signal amplification is strongly dependent on the conformation of the aptamer before and after binding to TLR. Strong signal is generated by completely unfolded aptamer binding to target TLR and low noise occurs in the presence of fully folded aptamer in the absence of target. Therefore, understanding how to obtain correct folding of aptamer is key to the detection process.

It has been surprisingly found that the receptorial affinity of NPs-APT depends on the distance of the aptamer from the nanoparticle nucleous. In particular, the use of polyamine spacer molecules such as for example SP and/or TP leads to the optimization of the binding capacity of targeted nanoparticles to plasmamembrane receptor decreasing the steric hindrance of the backbone polymer. Indeed, in case of direct binding of aptamer to nanoparticles the extent of cell targeting is significantly lower than using the spacer, probably because the spacer allows the right conformation for the ligand/recptor interaction.

The aptamers used in these sets of experiments are the following: R10-60 (TLR-9 aptamer, 5'-CCA GTC GTA CAG GAA ACA TGC GTT CTA GAT GTT CGG GGC-3') and PO R10-5 (aptamer without CpG, 5'-CCA GCC ACC TAC TCC ACC AGT GCC AGG ACT GCT TGA GGG G-3'),

Amperometric data in Table show that 1 h of incubation results in poor recognition and an SNR of only 1.5. Both the sample and blank control had a current in the range -200 to -400 nA. As the incubation time increased, the signal for sample TLR binding increased only in the presence of aptamer immobilized on polyaminated nanoparticles. After 24 h of incubation, the current of TLR reached -1986 nA, which is 6.6 times higher than that seen for the 1-h incubation. Meanwhile, as the sample signal increased between 1 h and 24 h of incubation, the background signal decreased from -223 nA to -53 nA and, following 24 h of incubation, the SNR was 41. Surprisingly, the binding of aptamer to nanoparticles in absence of a specific spacer (i.e., polyamines) did not show any significant difference between the current measured in the absence or in the presence of TLR.

**Table**

| | **A** | **B** | **C** | |
|---|---|---|---|---|
| Incubation Time (hours) | I/nA (polyaminated nanoparticles) | I/nA (untreated nanoparticles) | I/nA | SNR |
| 1 | -327±53 | -229±17 | -223±11 | A/C=1.5 B/C=1 |
| 3 | -1155±173 | -301±11 | -169±7 | A/C=6.8 B/C=1.7 |
| 5 | -1210±91 | -296±8 | -104±2 | A/C=11.6 B/C=2,8 |
| 24 | -2183±35 | 100±3 | -53±2 | A/C=41 B/C=1,8 |

| | | | | |
|---|---|---|---|---|
| A= is the current measured at a TLR concentration of 100 ng/ml in the presence of aptamer immobilizing polyaminated nanoparticles. B= is the current measured at a TLR concentration of 100 ng/ml in the presence of aptamer immobilizing nanoparticles in the absence of polyamination. C= is the current measured in the absence of TLR | | | | |

### Example 2

### Drug-loaded NPs formulation

Drug-loaded NPs containing a fluorescent dye such as 6-coumarin (fluorescent NPs), are formulated by using different methods such as a double emulsion-solvent evaporation technique. In a typical procedure, a solution of mesalazine (5-ASA) in water is emulsified with polymer (SP-PLGA or TP-PLGA) in chloroform containing 6-coumarin using a probe sonicator. 5-ASA is used as a model macromolecule in the formulation of fluorescent nanoparticles. The water-in-oil emulsion thus formed is further emulsified into aqueous solution of polyvinyl alcohol (PVA) used as an emulsifier by using sonication to form a multiple water-in-oil-in-water emulsion. The emulsion was stirred at room temperature to evaporate chloroform. Fluorescent NPs are recovered by ultracentrifugation, washed with double-distilled water, and then lyophilized to obtain a dry powder.

### Quantitation of 5-ASA

To quantify the amount of 5-ASA loaded in the nanoparticles, each sample is dissolved in DMF, and phosphate buffer solution (pH=8) was added up to 100 ml. The resulted system was stirred and filtered and the amount of drug in the filtrate was determined using a UV spectrophotometeric method at 331 nm.

### Calculation of 5-ASA loading efficiency

The loading efficiency of NPs was calculated according to the following equation:
Percentage of loading efficiency (LE %) = (ELDS / TLDS) × 100
Where ELDS is the empirical loaded drug in sample (the empirical measured amount of 5-ASA in the sample) and TLDS is the theoretical loaded drug in sample (theoretical amount of 5-ASA which should have been loaded in the sample in case of complete loading).
TLDS is calculated according to this equation: TLDS= WS/SRS, where WS is the weight of sample and SRS is the sum of polymer and 5-ASA weight ratios in the sample.

### In vitro drug release

Lyophilized drug-loaded NPs-APT are re-suspended in a flask containing either phosphate buffer (pH 7.4) or artificial intestinal fluid (according to US Pharmacopoeia 23) and incubated at 37°C under magnetic stirring. At appropriate intervals, 0.5 ml samples are withdrawn, centrifuged at 15,000 g for 30 min, and replaced by fresh buffer. Drug loads are determined by dispersing the same amount of NPs in 1M NaOH at 37°C until particles are completely degraded. The drug release is quantified by use of an isocratic high performance liquid chromatographic method developed to our specific requirements allowing detection of 5-ASA by UV absorbance at 340 nm. About 25% of the total initial amount of 5-ASA loaded onto the NPs is released into an intestinal fluid solution at pH 6.2 within 30 minutes. This release of 5-ASA from the NPs-ATP likely is owing to the release of 5-ASA near the surface of the NPs. After 30 minutes, the release kinetics of 5-ASA is more linear and about 35% of the total initial amount is released within 30 hours. This later 5-ASA release phase seems to be the result of 5-ASA diffusion across the NPs. Together, these results indicate that only free 5-ASA will be delivered rapidly (burst release) to the intestinal mucosa no more than 30 minutes after the 5-ASA-loaded NPs-APT come into contact with the intestinal fluid at colonic pH (pH 6.2).

### Encapsulation of 5-ASA-Loaded NPs-APT into Biomaterials

Chitosan powder is solubilized in acetic acid then neutralized by addition of NaOH. Medium-viscosity sodium alginate is prepared in NaCl. Alginate and chitosan solutions are mixed at a 1:1 ratio for a final concentration of 7 and 3 g/L, respectively. The polymer suspension is homogenized for 24 hours. This methodology has been already applied by others [Laroui et al., Gastroenterology. 2010, 138:3, 843-853.] Different NPs-APT containing a polyamine spacer according to the invention are added to obtain a 2-mg/mL concentration of hydrogel solution and stirred to disperse NPs throughout the polymer solution. A chelation solution containing 70 mmol/L of calcium chloride and 30 mmol/L of sodium sulfate is prepared. Kinetic of the swelling degree measured the evolution of water trapping in the hydrogel structure. Typically in an ionic hydrogel, the swelling degree evolution is inversely proportional to the power of ionic interactions between ions and polysaccharides. Thus, the higher swelling degree indicates the hydrogel is closer to collapsing. In the present study, we assessed the swelling degree of the biomaterial in solutions simulating gastric fluid at pH levels from 1 to 3 and simulating intestinal fluid at a pH level from 4 to 6. We observed that the hydrogel beads exposed to a gastric solution of pH 1, 2, or 3 for 24 hours are stable. However, the hydrogel beads exposed to the intestinal solution of pH 4 for 24 hours shrank and those exposed to a solution of pH 5 completely collapsed. In addition, we observed that the collapsing effect of the hydrogel beads in the intestinal solution is time-dependent. Together, these results indicate that this biomaterial could be used to deliver 5-ASA-loaded NPs to intestinal solutions of pH 5 and 6 (the approximate pH of the colonic fluid) but not at acidic pH values of 1, 2, or 3 (the approximate pH of the gastric fluid). In other words, these data indicate that the hydrogel composed of alginate and chitosan at a ratio of 7/3 (wt/wt) could be used to deliver 5-ASA-loaded NPs-APT according to the invention to the colon but not to the gastric gland.

### In Vitro Cytotoxicity

To assess potential toxicity of the 5-ASA-loaded NPs-APT according to the invention we used 2 different assays, the WST-1 assay and ECIS. For the WST-1 assay, Caco-2 and IEC6 cells are seeded in 96-well plates and grown in Dulbecco's Modified Eagle Medium. Cells are allowed to adhere for 24 h before medium was replaced with Opti-MEM (90µl per well) and exposed to several concentrations of chitosan/alginate-coated NPs-APT loaded with 5-ASA according to the invention, or alone for 24, 48, or 72 hours; the maximum concentration of NP-APT was 1 mg/mL. The WST-1 assay (Roche, Nutley, NJ) measures cleavage of the soluble, red tetrazolium salt WST-1 (4-[3-(4-iodophenyl)-2-(4-nitrophenyl)-2H-5-tetrazolio]-1,3-benzene disulfonate) by dehydrogenase present in intact mitochondria, which leads to formation of dark red formazan crystals. After assessing several NP-APT concentrations and exposure times, 10 µL of WST-1 proliferation reagent is added to Caco-2 or IEC6 cells (10 µL/well) and incubated for 1-2 hours at 37°C. The wavelength for measuring the absorbance of the formazan product was 440 nm. Cytotoxicity is expressed as a percentage of controls (untreated cells). 5-ASA-loaded NPs-APT according to the invention at a concentration of 1 mg/mL do not cause cellular death of Caco-2 cells over a 72-hour period compared with control materials. Similar results are found by using the normal intestinal enterocyte cell IEC6 cell line. For electrical impedance sensing (ECIS), the measurement system consists of an 8-well culture dish, the surface of which is seeded with cell cultures. Each well contains a small, active electrode (area, 5.10-4 cm²) and a large counter electrode (area, 0.15 cm²) on the bottom of each well. A lock-in amplifier, with an internal oscillator, relays a signal to switch between the different wells, and a personal computer controls the measurement and stores the data. Attachment and spreading of cells on the electrode surface change the impedance in such a way that morphologic information about the attached cells can be inferred. Caco-2 cells are seeded in ECIS plates coated with 10 µg/mL laminin I in Dulbecco's modified Eagle medium supplemented with 10% (vol/vol) heat-inactivated fetal bovine serum and 1.5 µg/mL plasmocin. Once cells reached confluence, NP-APT were added at a concentration of 1 mg/mL. Control cell cultures are used for each experiment; 5-ASA-loaded NPs-APT according to the invention are replaced by 400-nm latex microspheres. Basal resistance measurements are performed using the ideal frequency for Caco-2 cells, 500 Hz, and a voltage of 1 V. Even 80 hours of treatment with 5-ASA-loaded NPs-APT according to the invention did not affect the Caco2 monolayer resistance (before addition of 5-ASA-loaded NPs-APT of the invention, the resistance is 40,000 Ohms compared with 43,000 Ohms 80 hours after addition of 5-ASA-loaded NPs-APT of the invention). These data are supported by results with the control biocompatible latex beads (diameter, 200 nm) on the resistance of Caco-2 monolayers (40,000 vs 43,000 Ohms before and after administration of beads, respectively). Together, these results show that 5-ASA-loaded NPs-APT of the invention coated with chitosan/alginate do not affect cell growth or intestinal barrier function.

### Uptake Experiments

Dendritic cells (DCs) from rat intestine are used to investigate the uptake of NPs-APT containing a polyamine spacer according to the invention. Briefly, *Lamina Propria* digests are passed through 70- and 40-µm cell strainers; cells isolated are resuspended in iso-osmotic NycoPrep medium, and overlaid with RPMI 1640. The low-density fraction is collected after centrifugation at 1650 g for 15 min. Cells are washed and incubated with a mixture of mAb containing α-CD16/32 (2.4G2), 7-AAD viability staining solution, α-CD11c (HL-3), and α-MHCII (AF6-120.1), as well as the non-DC components α-DX5 (DX5), α-NK1.1 (PK136), and α-B220 (RA3-6B2). DCs are defined as CD11chiMHCII+ cells and non-DCs are excluded when sorted by flow cytometry on a FACSAria.

Fluorescent 5-ASA NPs-APT according to the invention are incubated with cells over a time course ranging from 15 min to 4 h (37°C, 5% CO₂). After cell/particle incubation, the cells are washed and detached by trypsinization. After centrifugation, cells are resuspended in a 0.4% trypan blue (TB) solution in Dulbecco's Phosphate Buffers Saline solution (D-PBS) to quench the extracellular FITC fluorescence. This assay is based on the observation that the vital dye TB, while quenching the FITC fluorescence of a noninternalized particle, causes them to fluoresce red whereas an internalized particle will fluoresce green. Cells are then centrifuged, the TB solution is removed, the cell pellet is resuspended in D-PBS, and sample is analyzed by flow cytometry (CyAn ADP, Dako), for green fluorescence. There are 10,000 cells measured in each sample. The kinetics of particle internalization is evaluated by using a flow cytometry method in which internalized particles are differentiated from membrane-bound particles with a trypan blue fluorescence quench. The time course of particle internalization is studied from 15 min to 4 h.

### Cellular internalization of Fluorescent 5-ASA NPs-APT using Confocal Laser Scanning Microcopy

DC cells are seeded in a T-25 flask in MEM supplemented with 1% FBS (low serum). Cells are then incubated for 4 h (37°C, 5% CO₂) with low serum MEM containing fluorescent labeled NPs-APT. The cells are resuspended in complete MEM containing 10% FBS, replated onto two 35-mm² glass bottom dishes. Nuclei are stained with DRAQ5 in complete MEM according to the manufacturer's protocol. DRAQ5 is a DNA-specific dye with far-red fluorescent properties (Ex: 647 nm, Em: 670 nm). AlexaFluor-555-labeledWGAin D-PBS (2.5 µ/ml; Molecular Probes) is used to visualize plasma membranes. Cells are then fixed with 4% paraformaldehyde. Microscopy is carried out on an confocal laser scanning microscope. Monitoring particle internalization by confocal microscopy gave a more complete understanding of particle intracellular localization. Confocal micrographs revealed that all internalized NPs-APT particles consistently migrated to the perinuclear region of the cells over time. Z series images were obtained and used to construct 3D representations that clearly show that nanoparticles are internalized by DC cells, and that the particles ultimately translocate to the perinuclear region of the cell. These data are consistent with intracellular translocation of either endosomal and/or lysosomal vesicles from the plasma membrane along microtubules in the minus direction toward the centrosome/microtubule-organizing center of the cell.

### Encapsulated 5-ASA-Loaded NPs-ATP reduce induced Colitis

Many experimental animal models have been used to study human IBD. One well-characterized animal model for the study of human ulcerative colitis is dextran sodium sulfate (DSS)-induced acute and chronic colitis with ulceration in mice, which is characterized by a marked loss in body weight, rectal bleeding, reduction in colon length, and destruction of the intestinal epithelium. We investigated the anti-inflammatory effects of ASA-loaded NPs-APT encapsulated in the hydrogel (alginate and chitosan: 7 g/L and 3 g/L, respectively) according to the invention in mice with active DSS-induced colitis. C57BL/6 mice received regular water and a gavage of 150 µL of hydrogel daily for 7 days (group 1), water with 3% DSS and gavage of 150 µL of hydrogel daily for 7 days (group 2), water with 3% DSS and gavage of 150 µL of hydrogel encapsulated with ASA-loaded NPs-APT of the invention daily for 7 days (group 3), water with 3% DSS and gavage of 150 µL of hydrogel encapsulated with NPs-APT of the invention daily for 7 days (group 4), water with 3% DSS and gavage of 150 µL of hydrogel containing free ASA daily for 7 days (group 5), water with 3% DSS and gavage of 150 µL of hydrogel encapsulated with dextran-FITC-loaded NPs daily for 7 days (group 6), and water with 3% DSS for 7 days (group 7). This treatment regimen results in intestinal colitis and histologic studies reveals DSS-induced cell-wall damage with epithelial exfoliation when compared with mice that receive water alone. In the DSS-treated mice, we also observed interstitial edema, dilation of vessels, and a general increase in the number of inflammatory cells (lymphocytes and polynuclear cells) in the lamina propria. To evaluate the role of ASA in the inflammatory process, encapsulated 5-ASA-loaded NPs-APT of the invention are administered by daily gavage for 7 days to the mice that also receive DSS in the drinking water; after 7 days the mice are killed. Our histologic studies shows reduced intestinal inflammation in mice with DSS-induced colitis treated with encapsulated 5-ASA-loaded NPs-APT of the invention, compared with control mice that receive only water. We additionally observe weight loss in the DSS-treated mice, which started at day 4 and reached a maximum at day 7. The administration of encapsulated 5-ASA-loaded NPs-APT of the invention reduces the weight loss of DSS-induced colitis. However, administration of 5-ASA to mice with DSS-induced colitis does not reduce the severity of weight loss compared with mice that receive DSS alone. Intestinal myeloperoxidase (MPO) activity is measured to indicate the extent of neutrophilic infiltration. MPO levels in the colon is increased significantly in mice with DSS-induced colitis compared with control mice that receive only water. Administration of encapsulated 5-ASA-loaded NPs-APT of the invention significantly reduces DSS-induced colonic inflammation, whereas administrations of biomaterial with free 5-ASA at an equivalent concentration has no effect on the basal MPO levels in the colonic mucosa. These results are reflected in the proinflammatory cytokine levels (TNF-α and IL-1β), which are measured by real-time reverse-transcription polymerase chain reaction in mucosa obtained from the colitic mice. As controls, biomaterials without or encapsulated with empty NPs did not have anti-inflammatory effects on DSS-induced colitis. Of particular interest is the internalization of particles into the immunocompetent/inflammatory cells within the inflamed region. The confocal micrographs revealed that all internalized NPs-APT particles of the invention consistently migrated to the perinuclear region of these cells over time. These data clearly shows the advantage of this vehicle that is able to drive the drug directly to the target cells possibly reducing the required dose and shortening the response time to therapies.

## Claims

1. Pharmaceutical composition for oral administration comprising:
a) a vector particle consisting of a material which resists proteolytic degradation, said particle encapsulating or retaining an active agent;
b) a binding moiety attached to the vector particle, wherein said binding moiety is able to specifically bind to cell receptors present in target cells which promote inflammatory and/or immunological response; and
c) a spacer molecule linking the vector particle and the binding moiety.

2. Composition according to claim 1, wherein the vector particle is a nanoparticle.

3. Composition according to claims 1-2, wherein the binding moiety comprises specific aptamers able to bind to Toll-like receptors, said aptamers being selected from the group consisting of single stranded RNA or double stranded DNA molecules.

4. Composition according to claim 3, wherein the Toll-like receptor is Toll-like receptor 9.

5. Composition according to claim 4, wherein the aptamer able to bind Toll-like receptor 9 is a single stranded DNA molecule selected from the group consisting of 5'-CCA GTC GTA CAG GAA ACA TGC GTT CTA GAT GTT CGG GGC-3', 5'-CCA GCC ACC TAC TCC ACC AGT GCC AGG ACT GCT TGA GGG G-3', 5'-TGA CTG TGA ACG TTC GAG ATG A-3' and CpG, 5'-TGA CTG TGA AGG TTG GAG ATG A-3'.

6. Composition according to claims 1-2, wherein the binding moiety comprises an antibody or a fragment thereof.

7. Composition according to claims 1-6, wherein the target cell is a human intestinal dendritic cell.

8. Composition according to claims 1-7, wherein the spacer molecule is a polyamine.

9. Composition according to claim 8, wherein the polyamine is spermine or tetraethylenepentaneamine.

10. Composition according to claims 1-9, wherein the nanoparticle comprises polyethylene glycol (PEG), poly(d,l-lactide-co-glycolide) or poly(d,l-lactide-co-glycolide) attached to spermine or tetraethylenepentaneamine.

11. Composition according to claims 1-10, wherein the active agent is selected from anti-inflammatories, immunomodulants, biocides, bactericides, antiviral agents, probiotics, nutraceuticals, enzymes, cytoprotectants, vaccines, polynucleotides, polypeptides and polysaccharides.

12. Composition according to claim 11, wherein the active agent is a drug that acts on gastrointestinal system or on immune system.

13. Composition according to claims 11-12, wherein the active agent is 5-aminosalicylic acid.

14. Composition according to claims 1-13, further comprising a coating protective matrix suitable for coating said vector particle attached to said binding molecule, said protective matrix being able to resist proteolytic degradation.

15. Composition according to claim 14, wherein the coating protective matrix suitable for coating the vector particle comprises chitosan and sodium alginate.

16. Composition according to claims 1-15, for the treatment of inflammatory diseases.

17. Composition or preparation according to claim 16, wherein said disease is inflammatory bowel disease.
